(19) 

**Europäisches Patentamt**
**European Patent Office**
**Office européen des brevets**

(11) **EP 4 511 847 B1**

(12) **EUROPEAN PATENT SPECIFICATION**

(45) Date of publication and mention
of the grant of the patent:
**22.10.2025 Bulletin 2025/43**

(21) Application number: **23720281.7**

(22) Date of filing: **19.04.2023**

(51) International Patent Classification (IPC):
*G16H 50/20* (2018.01)   *G16H 50/50* (2018.01)
*G16H 50/70* (2018.01)

(52) Cooperative Patent Classification (CPC):
**G16H 50/20; G16H 50/50; G16H 50/70**

(86) International application number:
**PCT/EP2023/060074**

(87) International publication number:
**WO 2023/203048 (26.10.2023 Gazette 2023/43)**

(54) **SYSTEM, METHOD AND COMPUTER PROGRAMS FOR ASSESSMENT OF BODY MOVEMENT'S CONDITIONS OR DISORDERS**

SYSTEM, VERFAHREN UND COMPUTERPROGRAMME ZUR BEWERTUNG VON KÖRPERBEWEGUNGSZUSTÄNDEN ODER -STÖRUNGEN

SYSTÈME, PROCÉDÉ ET PROGRAMMES INFORMATIQUES POUR L'ÉVALUATION DES AFFECTIONS OU DES TROUBLES DU MOUVEMENT CORPOREL

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **19.04.2022 EP 22382360**

(43) Date of publication of application:
**26.02.2025 Bulletin 2025/09**

(73) Proprietors:
• **Fundació Eurecat**
**08290 Cerdanyola del Vallès (ES)**
• **Hospital Sant Joan de Deu**
**08950 Esplugues de Llobregat (ES)**
• **Rigshospitalet**
**2100 Copenhagen Ø (DK)**
• **Asociación Duchenne Parent Project España**
**28045 Madrid (ES)**

(72) Inventors:
• **CLARAMUNT MOLET, Mireia**
**25008 Lleida (ES)**
• **IDELSOHN ZIELONKA, Sebastian**
**08014 Barcelona (ES)**
• **MIRALLES BARRACHINA, Felipe**
**08025 Barcelona (ES)**

• **MARÍ MARTÍNEZ, David**
**08012 Barcelona (ES)**
• **MIGLIORELLI FALCONE, Carolina Mercedes**
**08024 Barcelona (ES)**
• **GÓMEZ MARTÍNEZ, Meritxell**
**08028 Barcelona (ES)**
• **SUBÍAS BELTRÁN, Paula**
**08023 Barcelona (ES)**
• **ORTE DEL MOLINO, Silvia Julia**
**08912 Badalona (ES)**
• **CREMADES ROSELL, Xavier**
**08008 Barcelona (ES)**
• **MEDINA CANTILLO, Julita**
**08026 Barcelona (ES)**
• **VISSING, John**
**2150 Nordhavn (DK)**
• **MONTOLIO DEL OLMO, Maria Soledad**
**08024 Barcelona (ES)**

(74) Representative: **Torner, Juncosa I Associats, SL
C / Pau Claris, 108, 1r 1a
08009 Barcelona (ES)**

(56) References cited:
**WO-A1-2021/064195**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

• SCHOLLHORN W I ED - GEFEN AMIT ET AL: "Applications of artificial neural nets in clinical biomechanics", CLINICAL BIOMECHANICS, ELSEVIER, AMSTERDAM, NL, vol. 19, no. 9, 1 November 2004 (2004-11-01), pages 876 - 898, XP004593345, ISSN: 0268-0033, DOI: 10.1016/J.CLINBIOMECH.2004.04.005

• ALBARA AH RAMLI ET AL: "Gait Characterization in Duchenne Muscular Dystrophy (DMD) Using a Single-Sensor Accelerometer: Classical Machine Learning and Deep Learning Approaches", ARXIV.ORG, CORNELL UNIVERSITY LIBRARY, 201 OLIN LIBRARY CORNELL UNIVERSITY ITHACA, NY 14853, 31 July 2021 (2021-07-31), XP091004518

• ROMANO ALBERTO ET AL: "Evaluation of gait in Duchenne Muscular Dystrophy: Relation of 3D gait analysis to clinical assessment", NEUROMUSCULAR DISORDERS, ELSEVIER LTD, GB, vol. 29, no. 12, 5 November 2019 (2019-11-05), pages 920 - 929, XP085942427, ISSN: 0960-8966, [retrieved on 20191105], DOI: 10.1016/J.NMD.2019.10.007

**Description**

<u>Technical Field</u>

The present invention relates to a system, a method and computer programs for the assessment of body movement's conditions or disorders, particularly neuromuscular diseases or disorders, for instance, Muscular Dystrophy, Multiple Sclerosis, Amyotrophic lateral sclerosis (ALS), Myopathy, etc.

<u>Background of the Invention</u>

Neuromuscular disorders involve a range of conditions that impair the functioning of the muscles, either directly, being pathologies of the voluntary muscle, or indirectly, being pathologies of the peripheral nervous system or neuromuscular junctions. These disorders result in muscle weakness and fatigue that progress over time. Symptoms generally will depend on the type of neuromuscular disorder and the areas of the body that are affected.

Among such neuromuscular disorders, the Duchenne Muscular Dystrophy (DMD) is a rare, progressive, neuromuscular disease affecting about one in 5000 newborn boys. It occurs due to a gene mutation that inhibit the generation of the Dystrophin protein, causing muscle progressive degeneration. The disease affects mostly males, the symptoms start usually at age of 2-3 years old, and the diagnosis arrives on average at the age of 4 years old. The loss of muscle implies a progressive weakness that starts in lower limbs and affects severely the gait capacity. Becker Muscular Dystrophy (BMD), is a less severe and frequent condition (one-third of DMD) affecting the same gene, being the Dystrophin manufactured but not in the correct amount or quality. In the near future new therapies are expected to fight more efficiently against the disease evolution.

In this sense, there is an increasing interest in digitalizing and objectivizing the standard methodology that is currently used to monitor the evolution of the neuromuscular disorders or diseases. Leading to relevant clinical endpoints, that can assist and improve, not only the quantity of data, but also the quality of this data.

To monitor and follow-up the evolution of different chronic diseases different function tests can be used. One of such test is the Six Minute-Walk Test (6MWT), which is specifically widely implemented for neuromuscular disorders assessment in clinical practice and clinical trials. Nevertheless, other shorter/longer versions of the test or alternative function tests can be also used.

WO2021064195-A1 relates to biomarkers and to sensor-diagnostic and monitoring technology for diseases including neuromuscular/degenerative, neurological, neurodegenerative or polygenic diseases or disorder. In particular, this international patent application proposes a method for identifying a biomarker of interest for a disease by determining a candidate biomarker, obtaining from at least one motion sensor first motion data from at least one person having the disease, obtaining from the at least one motion sensor second motion data from at least one person not having the disease, calculating at least a first value related to the candidate biomarker based on the first motion data and at least a second value related to the candidate biomarker based on the second motion data; in a first determination step, determining based on the first and the second values of the candidate biomarker whether the candidate biomarker distinguishes between the at least one person having the disease and the at least one person not having the disease, obtaining first clinical score data according to a conventional clinical score protocol from at least one person having the disease, obtaining second clinical score data according to the conventional clinical score protocol from at least one person not having the disease, determining a level of correlation between a set of the calculated first and second values of the candidate biomarker and a set of the obtained first and second clinical score data, identifying, based on the result of the first determination step and on the level of correlation, whether the candidate biomarker is a biomarker of interest.

Scientific document "Applications of artificial neural nets in clinical biomechanics" Schollhorn W I ED-Gefen Amit Et al. provides an overview of current applications of artificial neural networks in the area of clinical biomechanics.

There is a need for more clinical endpoints that can relate to the functional evolution of movement's conditions or disorders, independently of the user collaboration during the test.

<u>Description of the Invention</u>

Present invention provides, with a patient-centric focus, the measurement of a set of biomechanical and/or physiological variables that are combined using statistical analysis and machine learning techniques to generate a new score that correlates, in a more reliable and responsive way, to the evolution of body movement's conditions or disorders of a patient, in particular the evolution of neuromuscular disorders/diseases. These new score will improve the current functional tests in precision and duration and so creating a positive impact on the diagnosis.

The invention is particularly applicable for Duchenne Muscular Dystrophy (DMD) or neuromuscular diseases, not limitative, as the invention can be applied to other conditions or pathologies where the mobility is altered in some way. Thus, in one embodiment the body movement's condition or disorder is DMD. In other embodiments, the body movement's condition or disorder is a neuromuscular disorder or disease. Particularly, the body movement condition or disorder is for example Becker, Myotonic dystrophy, Glycogen storage disease type II, also called Pompe disease, or even to neuropathies such as Chronic Inflammatory Demyelinating Polyneuropathy (CIDP), Charcot-Marie-Tooth, Guillain-Barré or Anti-myelin-associated-glycoprotein (anti-MAG) neuropathy.

To that end, embodiments of the present invention disclose, according to a first aspect, a system for assessment of body movement's conditions or disorders. The system comprises several monitoring sensors, a memory or database and a processing unit.

The monitoring sensors are adapted and configured to be attached to a different area of the body of a person to obtain one or more variables of said area according to a specified sensor configuration. The variables including biomechanical and/or physiological variables.

The monitoring sensors comprise at least three sensors that are selected from the group consisting of: a heart rate sensor, an inertial sensor or a goniometer, and a plantar pressure sensor. Additional monitoring sensors could be added to the system to complement the current ones.

The memory or database can have stored therein control variables that are obtained, during different sessions of a same or different duration, from healthy users while they performed a function test using the monitoring sensors. In some embodiments, the memory or database can have stored therein pathology variables that are obtained, during different sessions of a same or different duration, from unhealthy users while they performed the function test using the monitoring sensors, the unhealthy users suffering a given body movement condition or disorder. In yet some embodiments, the memory or database can have stored both the control variables and the pathology variables. In this latter case, the session conditions for the healthy and unhealthy users are the same. In either case, both the control and the pathology variables, for each session, are stored classified in at least three different categories, including cardiac, kinematics and plantar-pressure.

The processing unit is configured to: generate a normality model for said given body movement condition or disorder by implementing a statistical-based feature selection process on the stored variables (i.e. the control, the pathological or both), where this generated normality model defines a unified category score for each category of said at least three different categories and where each unified category score outlines the variables that better characterize the given body movement condition or disorder; get/receive/acquire variables obtained from a given user while the given user performed the function test during a given session using the monitoring sensors, the given user suffering the given body movement condition or disorder; classify the gotten/received/acquired variables of the given user into the at least three different categories, and select, for each category, the variables of the given user that are significant by considering the variables that better characterize the given body movement condition or disorder from the generated normality model; compute, for the selected variables of the given user, a unified category score for each category; and compute a condition or disorder score for the given user as the deviation between the computed unified category scores of the given user with the generated normality model.

Embodiments of the present invention also disclose, according to a second aspect, a (computed-implemented) method for assessment of body movement's conditions or disorders, wherein a memory or database comprises stored therein at least one of: control variables obtained from healthy users while they performed a function test using several monitoring sensors during different sessions of a same or different duration, and pathology variables obtained from unhealthy users while they performed the function test using the several monitoring sensors during different sessions of a same or different duration, the unhealthy users suffering a given body movement condition or disorder, each one of the different monitoring sensors, for each session and for each healthy user and/or unhealthy user, being adapted and configured to be attached to a different area of the body to obtain one or more biomechanical and/or physiological variables of said area according to a specified sensor configuration. In case both control and pathology variables are stored in the database the session conditions for both types of users are the same.

According to the proposed method, the control and/or the pathology variables, for each session, are stored classified in at least three different categories, including cardiac, kinematics and plantar-pressure. Likewise, a processing unit via one or more processors, comprises: generating a normality model for said given body movement condition or disorder by implementing a statistical-based feature selection process on the stored variables, the generated normality model defining a unified category score for each category of said at least three different categories, each unified category score outlining the variables that better characterize the given body movement condition or disorder; getting/receiving/acquiring, once stored in the memory or database, variables obtained from a given user while the given user performed the function test during a given session using the monitoring sensors, the given user suffering the given body movement condition or disorder; classifying the gotten/received/acquired variables of the given user into the at least three different categories, and selecting, for each category, the variables of the given user that are significant by considering the variables that better characterize the given body movement condition or disorder from the generated normality model; computing, for the selected variables of the given user, a unified category score for each category; and computing a condition or disorder score for the given user as the deviation between the computed unified category scores of the given user with the generated normality model.

The function test can consist in performing easy physical exercises such as moving the arms or getting up and sitting down from a seat, among others. In other embodiments, the function test comprises other more difficult tests such as the 6-Minute Walking Test, the 10-Meter Walking Test, the Timed Up and Go Test, the Stair Climb Test, etc. In a particular embodiment, the function test comprises the 6-Minute Walking Test.

According to the invention, the specified sensor configuration comprises a time-synchronization with the other monitoring sensors.

In an embodiment, the different categories comprise five categories comprising cardiac, kinematics, plantar-pressure, spatial-temporal and electromyography. In this case, the monitoring sensors can be selected from the group consisting of a heart rate sensor, an electromyography sensor, an inertial sensor or a goniometer, and a plantar pressure sensor.

It should be noted that in other embodiments, the different categories could comprise clinical parameters instead of or complementarily to the above-mentioned technical parameters (i.e. cardiac, kinematics, plantar-pressure, etc.). Some examples of clinical parameters are proximal/distal weakness, equilibrium/stability; asymmetry, stiff joints, steppage/ataxia, etc.

In some embodiments, the different categories considered can be given a same or a different weight. That is, depending on the specific body movement condition or disorder to be evaluated some of the categories can be provided with a higher weight (meaning that such specific category/categories is/are more relevant for that specific condition or disorder) than the other category/categories.

In an embodiment, the memory or database stores both the control variables and the pathology variables. In this embodiment, the statistical-based feature selection process comprises: comparing the variables between two different sessions and considering the control variables that exhibited a same underlying distribution for both sessions as robust control variables; and comparing the pathology variables between two different sessions and considering the pathology variables that exhibited a same underlying distribution for both sessions as robust pathology variables.

Moreover, the statistical-based feature selection process particularly further compares the robust control variables with the robust pathology variables, obtaining a compared set of robust variables as a result, and selects from the compared set, the variables showing a difference lower than a given threshold as the variables that better characterize the given body movement condition or disorder.

In an embodiment, the memory or database simply stores one of the control or pathology variables. In this case, the statistical-based feature selection process comprises comparing the stored (control or pathology) variables between two different sessions and considering the (control or pathology) variables that exhibited a same underlying distribution for both sessions as robust (control or pathology) variables.

According to the invention, additional mobility metrics (e.g. the North Star Ambulatory Assessment (NSAA), Performance of Upper Limb (PUL), Medical Research Council (MRC), Tinetti, Muscular Impairment Rating Scale (MIRS), Vignos Lower Extremity Scale (VIGNOS), among others) can be considered to improve the assessment. Therefore, in some embodiments, the memory or database further stores one or more control mobility metrics and one or more pathology mobility metrics obtained from the healthy and unhealthy users, respectively, during said different sessions, the method further comprising validating the computed condition or disorder score by comparing it with a normalized score obtained from both the one or more control mobility metrics and the one or more pathology mobility metrics. Note that in the case that only one of the healthy or unhealthy groups is considered the mobility metrics will be computed for such specific group only.

In some embodiments, the processing unit can advantageously further comprise updating the generated normality model with the (biomechanical and/or physiological) variables of the given user. Consequently, the normality model can be continuously trained as new tests/patients are considered.

Embodiments of the present invention also disclose a (computed-implemented) method for assessment of body movement's conditions or disorders, wherein a memory or database comprises stored therein a normality model (particularly generated as explained before) for a given body movement condition or disorder. According to this method, once a new user suffering the given body movement condition or disorder or once the given user, but for a new performance/session, has to be assessed, the method comprises performing by one or more processors of a processing unit: getting/receiving/acquiring, particularly once stored in the memory or database, biomechanical and/or physiological variables obtained from said (new or given) user while (s)he performed the function test during a session using the monitoring sensors; classifying the gotten/receive/acquired variables of the (new or given) user into the at least three different categories, and selecting, for each category, the variables that are significant by considering the variables that better characterize the given body movement condition or disorder from the stored normality model; computing, for the selected variables of the (new or given) user, a unified category score for each category; and computing a condition or disorder score for the (new or given) user as the deviation between the computed unified category scores of the user with the stored normality model.

It should be noted that in some embodiments the getting/receiving/acquiring step is performed in real time, i.e. before previously storing the (biomechanical and/or physiological) variables.

The invention in the generating step can advantageously further comprise defining an n-dimensional Gaussian model using each unified category score and the control variables, where each dimension of the n-dimensional Gaussian model corresponds to one of the categories.

The processing unit can be arranged/located on a computer device such as a PC, a laptop, a smartphone, a tablet, etc. or in the cloud. The memory/database can be local to the processing unit or remote thereof. If remote, communication between both components is particularly wireless. The communication between the monitoring sensors and the memory/database is also particularly wireless.

Other embodiments of the invention that are disclosed herein also include software programs to perform the method embodiment steps and operations summarized above and disclosed in detail below. More particularly, a computer program product is one embodiment that has a computer-readable medium including computer program instructions encoded thereon that when executed on at least one processor in a computer system causes the processor to perform the operations indicated herein as embodiments of the invention.

Brief Description of the Drawings

The previous and other advantages and features will be more fully understood from the following detailed description of embodiments, with reference to the attached figures, which must be considered in an illustrative and non-limiting manner, in which:

Fig. 1 is a flow chart of a method for assessment of body movement's conditions or disorders, according to an embodiment of the present invention.

Fig. 2 is a flow chart illustrating how the normality model for a given condition or disorder is generated, according to an embodiment of the present invention.

Fig. 3 is a boxplot distribution for each one of the assessed categories. Control group achieves scores closer to one while DMD group achieves lower scores.

Fig. 4 are spider plots of the computed scores for a control subject and 5 DMD patients with different levels of affection. While NSAA scores decrease, so do the scores. However, these reductions are not uniform among categories, being highly dependent on the subjects.

Fig. 5 illustrates the accuracies of 6- and 2-minutes scores, 6MWT and 2MWT in comparison with NSAA score.

Fig. 6 illustrates the regression with the target variables of each score methodology with age.

Detailed Description of the Invention and of Particular Embodiments

Present invention provides a new e-health platform/solution for assessment of body movement's conditions or disorders, particularly neuromuscular diseases or disorders. The present invention may be also applied for the assessment of other diseases/disorders or even neuropathies.

The invention integrates various (portable) monitoring sensors (e.g. heart rate, electromyography, inertial or goniometer, plantar pressure) to acquire different biomechanical and/or physiological parameters/variables during the performance of functional tests (e.g. 6-Minute Walking Test, 10-Meter Walking Test, Timed Up and Go Test, Stair Climb Test, moving the arms, getting up and sitting down from a seat, etc.). The monitoring sensors are attached to different areas of the body of a subject to obtain biomechanical and/or physiological variables of said area. Particularly, the different monitoring sensors are synchronized.

The different parameters/variables obtained are then stored and processed and the evolution of such conditions or disorders computed by assessing the functional capacity of the patient/user with long-lasting conditions such as, among others, muscular disturbances, respiratory diseases, etc.

When a test is finished, the different parameters obtained can be transmitted either to a software application (APP) run in a portable computing device such as a smartphone or a tablet, among others, or to a computer/server (e.g. a platform backend). In some embodiments, the different parameters can be stored in a database. If transmitted to the APP the latter can further transmit the session data to the platform backend for the session data be stored (in a memory of the platform), processed and analyzed. In some embodiments, the processing and analyzing of the session data is directly made by the APP.

The APP can be used by healthcare professionals to guide them through all the steps to facilitate the process of connecting the monitoring sensors and performing each function test. Either the platform or the APP can show the final assessment performed.

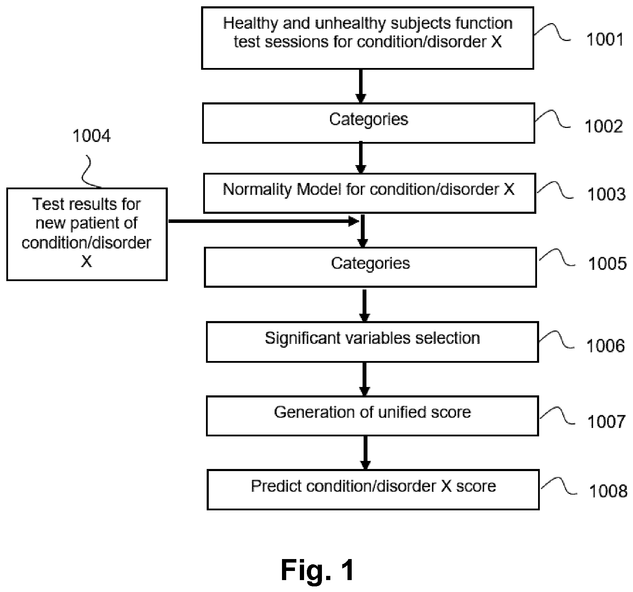

Referring to Fig. 1, the assessment of the body condition or disorder "X" comprises, at step 1001, the execution of the function tests, during different sessions of a the same or different extent, by the control group and the pathology group to obtain biomechanical and/or physiological variables thereof using the cited monitoring sensors. At step 1002, the control and the pathology biomechanical and/or physiological variables, for each session, are classified in different categories. In some embodiments, such classification is made by considering three categories, particularly cardiac, kinematics and plantar-pressure. In some embodiments, the classification is made by considering additional categories, for instance four, five, six, etc. In some particular embodiments, five categories are applied, including cardiac, spatial-temporal, electromyography, kinematics and plantar-pressure.

In yet other alternative or complementary embodiments, the classification is made by considering clinical parameters such as steppage/ataxia, proximal/distal weakness, equilibrium/stability; asymmetry, stiff joints, among others.

Once said categorization of the variables is made, at step 1003, a normality model of the body movement condition or disorder "X" is generated. This can be done by implementing a statistical-based feature selection process over the control and pathology biomechanical and/or physiological variables that involves the calculation of a unified category score for each category outlining the biomechanical and/or physiological variables that better characterize the body movement condition or disorder "X". This possibility to divide the score into the different categories, obtaining multicategory score, eases treatment personalization.

In other embodiments of the invention, not illustrated, the assessment can be made by considering just one of the groups, i.e. the control or the pathology. In this alternative case, the normality model will be generated by executing the statistical-based feature selection process using the variables obtained from one of the groups only.

In some embodiments, machine learning algorithms are used in said statistical-based feature selection process.

Continuing with the explanation of Fig. 1, when the test results (i.e. biomechanical and/or physiological variables) of a new patient of condition or disorder "X" arrive or are obtained (step 1004), these are classified, step 1005, into the same categories applied for the control and pathology groups. Then, at step 1006, for each category applied, the significant biomechanical and/or physiological variables of the new patient are selected and for the latter a unified category score computed (step 1007) for each category. Finally, at step 1008, a score for condition or disorder "X" is computed for the new patient. This can be done by calculating the deviation of the computed unified score from the normality model.

The final score aims to be an indicator that resumes the overall performance of the function test. An indicator that is reliable and responsive to the function test and that can be used as an endpoint for the time evolution of the condition/disorder and for clinical trials.

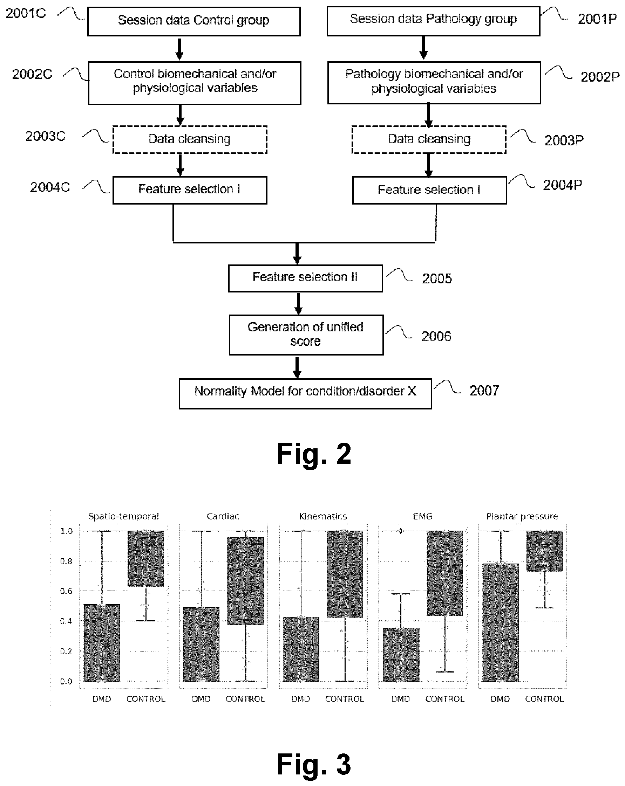

Referring to Fig. 2, an embodiment of the normality model generation process is illustrated. Similar to the embodiment of Fig. 1, in this case both control and pathology groups are also considered. According to this particular embodiment, both the control and pathology biomechanical and/or physiological variables (2002C, 2002P) obtained during the different sessions (2001C, 2001P) can be first (optionally) cleansed (steps 2003C, 2003P).

Then, the statistical-based feature selection process is implemented. At step 2004C, a first feature selection process is executed to select the most robust, and repeated, variables. At step 2004P an equivalent or similar process for the pathology group is made. This first feature selection process can be done by comparing the (control/pathology) biomechanical and/or physiological variables between two different sessions. The (control/pathology) biomechanical and/or physiological variables exhibiting the same underlying distribution for both sessions are considered robust.

At step 2005, a second feature selection process is executed. This can be done by comparing the robust control biomechanical and/or physiological variables (obtained in 2004C) with the robust pathology biomechanical and/or physiological variables (obtained in 2004P), and by selecting from the compared set, the biomechanical and/or physiological variables showing a difference lower than a given threshold. Finally, at steps 2006 and 2007, a unified score for each category is computed and the normality model generated.

Following a particular example of the application of the present invention for the assessment of DMD is detailed.

A pilot study was conducted on 28 male children (age 6.3-15.4 years) with DMD and 2 male children with (Becker Muscular Dystrophy) BMD (age 17.0-17.9 years) who attended their control visits to the Rehabilitation Service of the Sant Joan de Déu Hospital (Barcelona, Spain). Most of the patients were treated with corticoids. The control group consisted of 27 healthy male children (age 9.0-13.9 years) that attended the *Maristes la Inmaculada School (Barcelona).* As DMD affects mainly males, all tests were performed with males.

Performed Tests

The 6MWT was carried out on a standardized, level, undisturbed, and well-lit hallway that measured 25 meters. Two cones were used to define the start and the end of the walkway. Prior to each test, surface EMGs were calibrated by having the participant perform a maximal voluntary contraction of the hamstrings, quadriceps, and gluteus muscles. Furthermore, the insoles were also calibrated. All calibration was done according to a standard procedure set on the proposed system. The participants were instructed to walk as fast as possible, without running, in a straight line back and forth between two cones for 6 minutes. They were permitted to rest if needed, by leaning against the wall. Stopwatch did not stop during rest period.

The completed distance was measured at two minutes (2MWT) and at 6 minutes.

The 6MWT test was conducted on three sessions:

- Session 1 (t0). The first session, tagged as "t0", was aimed at collecting baseline data. During this session, the participant performed the 6MWT once.

- Session 2 (t0r). The goal of the second session was to determine the test-retest reliability and was tagged as "t0r". A subgroup of participants performed the test with a time gap of approximately 3-4 weeks.

- Session 3 (t1). The third session was tagged as "t1" and was planned to validate the responsiveness. The test was performed with a time gap of approximately 6 months.

During the sessions, the monitoring sensors were attached to different areas of the body to measure the biomechanics/physics of the 6MWT. The type of attached sensors and their information is summarized in Table 1. The list of all acquired variables was grouped into five categories: cardiac, spatial-temporal, electromyography, kinematics and plantar-pressure.

**Table 1.**

| Sensor type | Description | Category |
|---|---|---|
| Heart rate | A strap applied below the chest, to measure cardiorespiratory parameters. | Cardiac |
| Surface EMGs | EMGs were integrated in custom made, size-appropriate shorts that enabled recording of muscle activity. | EMG |
| Inertial sensors | Sensors were placed at the thigh, shank, and in the plantar insoles to measure joint kinematics. | spatial-temporal, kinematics |
| Plantar pressure | Pressure sensors were integrated in insoles which were size appropriate and used to obtain information on weight distribution during gait. | Plantar pressure, spatial-temporal, kinematics |

Furthermore, in each of the sessions, clinicians computed the North Star Ambulatory Assessment (NSAA), a scale used to measure functional motor abilities, for DMD patients. Control subjects received the maximum NSAA score. Subjects with medium NSAA values (from 15 to 20) at t0 suffered a steep decline between t0 and t1. The distance for 6MWT (6MWTD) was also computed. These two metrics (NSAA and 6MWTD) were used for posterior comparison with the computed score. These measurements were evaluated on a time window of six minutes, and on a time window of two minutes. The data analysis pipeline was executed for both time-windows.

Data Analysis

To obtain a multidimensional score that takes into consideration the information classified into the five listed categories the method was divided in two stages. The purpose of the first stage is the selection of the most robust variables which in turn present significantly different values between control and pathology subjects. These variables will be finally united into one representative variable for each of the categories. The objective of the second stage is to generate a normality model (or *"physiological walking model"*) composed of the five categories. The final score for a specific pathological subject is subsequently determined by analysing the distance of its variables to the normality model.

First stage:

The purpose of this stage was to discard the variables that were not useful for discriminating pathologic and normal behaviours. It involved the following sub-stages:

- Data cleansing. Due to the nature of the DMD disorder, it is considered that the affectation is almost symmetrical between the right and left limbs. Thus, the variables corresponding to the sensors placed in both right and left parts of the body were merged into a single measure representing its mean value.

- Robust variable selection. The most robust and repeatable variables were selected considering the t0 and t0r sessions for control and DMD subjects. The most robust variables were expected to have similar values between these two sessions as they were recorded in a short time-window. As the sets of data to compare arise from the same individuals at different sessions, t0 and t0r, the data was paired. To determine which statistical test was the most suitable for each variable, a Shapiro-Wilk test was applied. If the variable followed a normal distribution for both the t0 sessions set and the t0r sessions set, a Paired Sample T-Test was applied. If this condition was not met, the chosen test was Wilcoxon Signed-Rank Test. The variables that exhibited the same underlying distribution for both sessions ($p > 0.05$) were considered robust and used for subsequent analysis.
- Selection of the variables that best characterize the condition/disorder. To identify the variables that characterize the pathology, this step compares how the evolution of the data differs between the control group and the pathology group. To do so, a Kruskal-Wallis test for unpaired data was used to compare the values of each one of the variables between control and pathology group. The values that shown a significant difference between groups (threshold $p<0.01$) were selected for further analysis.
- Generation of a unified score for each one of the categories. The selected variables, which belonged to one of the five categories, were firstly normalized (being 0 the minimum and 1 the maximum value). If the control median value was lower than the pathology median value, the variable was inverted to have a lower DMD score than a control score. Finally, the unified variable for each of the categories was computed as the mean of the normalized values of that category and normalized between 0 and 1.

Second stage

While the determination of a category-by-category score may be useful to assess the subject performance in different aspects of the gait, there is interest in the definition of a score that characterizes the decline of the subject in the moment of the evaluation. The purpose of this stage was to determine the normality model and subsequent condition or disorder score (or pathology score) for one or more DMD subjects.

- Generation of the normality model. The unified variable for each of the categories and for the control group was further used to define a 5-dimensional gaussian model, where each dimension corresponded to one of the categories.
- Obtention of the pathology score. Each DMD and control subject obtains a score. The score was computed as the weighted log probability of belonging to the normality model. If the probability is the highest (which is expected for the control subjects) the score is 1. The lower the probability, the further the subject is from normality, and therefore has a greater impairment.

Score validation

The computed pathology score (6M+ and 2M+ for 6 and 2 minutes, respectively) was validated by comparing it with the normalized NSAA score (from 0 to 1). For further comparisons, the 6MWTD and 2MWTD were also normalized and compared with NSAA. For the four measurements, accuracy was computed as:

$$100*(1-|score\text{-}p - score\text{-}v|)$$

Where score-p and score-v represented the predicted score and the NSAA normalized score, respectively.

Finally, as DMD produces a progressive muscle degeneration, it is expected to obtain worse performance while age increase. To evaluate this effect, the linear regression between age and NSAA, the predicted scores (2M+ and 6M+), and the 6MWT and 2MWT distances was computed.

**Results**

Generation of a unified score for each category

The measurements of the 5 categories were assessed for 2- and 6-minutes intervals, obtaining a unified score for each category and for each period. Fig. 3 show as an example, the distribution of each one of the unified variables for both control an DMD groups, for 6 minutes.

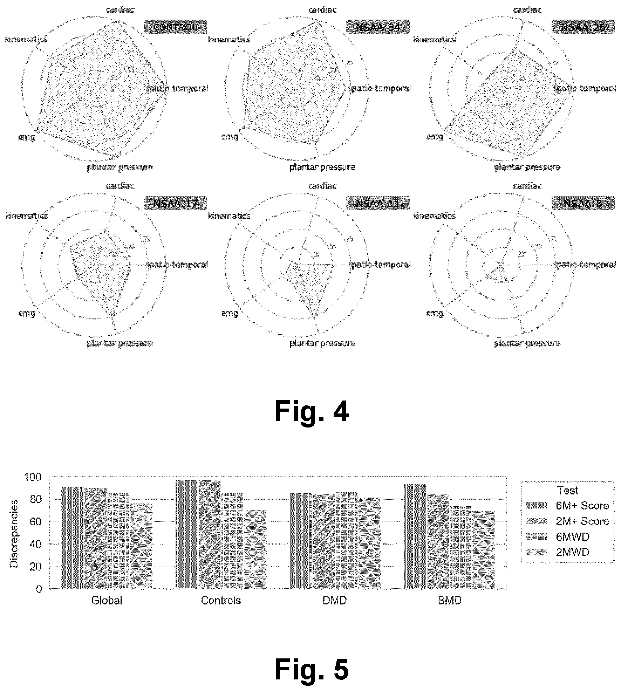

The generation of these categorical scores allow to represent the deviations of the pathological subjects in diverse fields related to normal gait. As an example, Fig. 4 shows how different DMD patients presented different performances in the evaluated categories.

The pathology 2M+ and 6M+ score and its comparison with NSAA and distances.

To validate the computed score, the NSAA scale was taken into consideration as the gold-standard. Fig. 5 shows the global accuracy of the pathology scores (2M+ and 6M+) and 6MWTD and 2MWTD in comparison with NSAA. 6-minutes and 2-minutes test presented the highest accuracies for controls. For DMD, 6M+ and 6MWTD performances were similar (86.00 and 86.30 respectively). While the performance of 2M+ in comparison with 2MWTD was slightly higher (85.03 and 79.68 respectively). The highest decreases were observed in BMD, with a drop in performance of 19 points for 6M+ vs. 6MWTD and a drop of 15 points for 2M+/2MWTD.

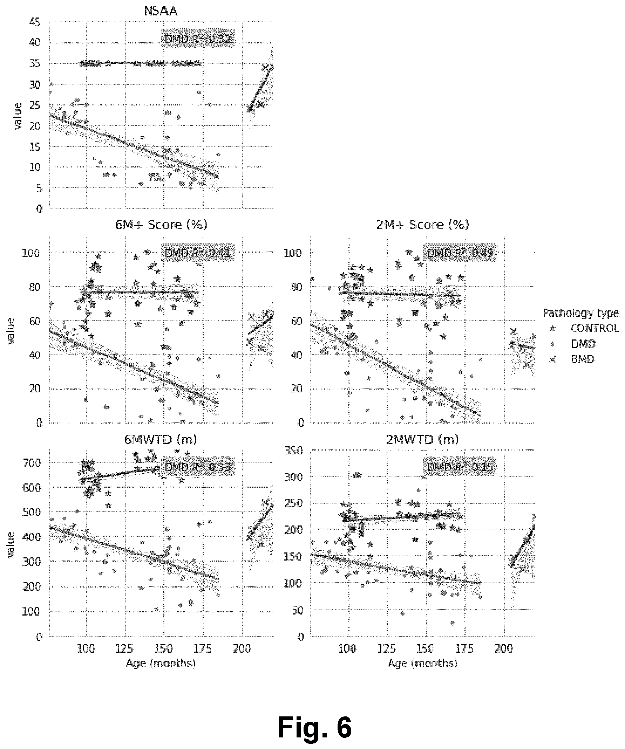

Finally, the regression with the ages of the patients is shown in Fig. 6. As a progressive disorder, it is expected to have a negative correlation with age. 6M+ and 2M+ scores were the most negatively correlated with age ($R^2$ of 0.41 and 0.49 respectively), with higher values than NSAA score.

The present invention has been described in particular detail with respect to specific possible embodiments. Those of skill in the art will appreciate that the invention may be practiced in other embodiments. For example, the nomenclature used for components, capitalization of component designations and terms, the attributes, data structures, or any other programming or structural aspect is not significant, mandatory, or limiting, and the mechanisms that implement the invention or its features can have various different names, formats, and/or protocols. Further, the system and/or functionality of the invention may be implemented via various combinations of software and hardware, as described, or entirely in software elements. Also, particular divisions of functionality between the various components described herein are merely exemplary, and not mandatory or significant. Consequently, functions performed by a single component may, in other embodiments, be performed by multiple components, and functions performed by multiple components may, in other embodiments, be performed by a single component.

Certain aspects of the present invention include process steps or operations and instructions described herein in an algorithmic and/or algorithmic-like form. It should be noted that the process steps and/or operations and instructions of the present invention can be embodied in software, firmware, and/or hardware, and when embodied in software, can be downloaded to reside on and be operated from different platforms used by real-time network operating systems.

The scope of the present invention is defined in the following set of claims.

**Claims**

1.  A system for assessment of body movement's conditions or disorders, comprising:

    several monitoring sensors, each one being adapted and configured to be attached to a different area of the body of a person to obtain one or more variables of said area according to a specified sensor configuration, the one or more variables including at least one of biomechanical and physiological variables;
    a memory or database, having stored therein at least one of:

       control variables, obtained during different sessions of a same or different duration from healthy users while they performed a function test using the monitoring sensors, and
       pathology variables, obtained during different sessions of a same or different duration from unhealthy users while they performed the function test using the monitoring sensors, the unhealthy users suffering a given body movement condition or disorder; and

    a processing unit operatively connected to said memory or database;

    **characterized in that**:

    the monitoring sensors comprise at least three sensors that are selected from the group consisting of: a heart rate sensor, an inertial sensor or a goniometer, and a plantar pressure sensor;
    the control variables, the pathology variables, or both, for each session, are stored classified in at least three different categories, including cardiac, kinematics and plantar-pressure; and
    the processing unit is configured to:

       generate a normality model for said given body movement condition or disorder by implementing a statistical-based feature selection process on the stored control and/or pathology variables, the generated normality model defining a unified category score for each category of said at least three different categories, each unified category score outlining the variables that better characterize the given body movement condition or disorder;

get variables obtained from a given user while the given user performed the function test during a given session using the monitoring sensors, the given user suffering the given body movement condition or disorder;

classify the gotten variables of the given user into the at least three different categories, and select, for each category, the variables of the given user that are significant by considering the variables that better characterize the given body movement condition or disorder from the generated normality model;

compute, for the selected variables of the given user, a unified category score for each category; and compute a condition or disorder score for the given user as the deviation between the computed unified category scores of the given user with the generated normality model.

2. The system of claim 1, wherein the at least three different categories comprise two additional categories, including spatial-temporal and electromyography, and wherein the monitoring sensors are selected from the group consisting of: a heart rate sensor, an electromyography sensor, an inertial sensor or a goniometer, and a plantar pressure sensor.

3. The system of any one of the previous claims, wherein the memory or database is configured to store both the control variables and the pathology variables, and wherein the statistical-based feature selection process comprises:

comparing the control variables between two different sessions and considering the control variables that exhibited a same underlying distribution for both sessions as robust control variables; and

comparing the pathology variables between two different sessions and considering the pathology variables that exhibited a same underlying distribution for both sessions as robust pathology variables.

4. The system of claim 3, wherein the statistical-based feature selection process further comprises comparing the robust control variables with the robust pathology variables, obtaining a compared set of robust variables as a result, and selecting from the compared set, the variables showing a difference lower than a given threshold as the variables that better characterize the given body movement condition or disorder.

5. The system of any one of the previous claims, wherein the specified sensor configuration comprises a time-synchronization with the other monitoring sensors.

6. The system of any one of the previous claims, wherein the function test comprises at least one of: moving the arms, getting up and sitting down from a seat, a 6-Minute Walking Test, a 10-Meter Walking Test, a Timed Up and Go Test, and a Stair Climb Test.

7. A computed-implemented method for assessment of body movement's conditions or disorders, wherein a memory or database comprises stored therein at least one of:

control variables, obtained from healthy users while they performed a function test using several monitoring sensors during different sessions of a same or different duration, and

pathology variables, obtained from unhealthy users while they performed the function test using the several monitoring sensors during different sessions of a same or different duration, the unhealthy users suffering a given body movement condition or disorder,

each one of the different monitoring sensors, for each session and for each healthy and unhealthy user, being adapted and configured to be attached to a different area of the body to obtain one or more variables of said area according to a specified sensor configuration, the one or more variables including at least one of biomechanical and physiological variables;

**characterized in that**:

the monitoring sensors comprise at least three sensors that are selected from the group consisting of: a heart rate sensor, an inertial sensor or a goniometer, and a plantar pressure sensor;

the control variables, the pathology variables, or both, for each session, are stored classified in at least three different categories, including cardiac, kinematics and plantar-pressure;

and **in that** the method comprises performing by one or more processors of a processing unit the following steps:

generating a normality model for said given body movement condition or disorder by implementing a statistical-based feature selection process on the stored control and/or pathology variables, the generated normality model defining a unified category score for each category of said at least three different categories, each unified category score outlining the variables that better characterize the given body movement condition or disorder;

getting, once stored in the memory or database, variables obtained from a given user while the given user performed the function test during a given session using the monitoring sensors, the given user suffering the given body movement condition or disorder;

classifying the gotten variables of the given user into the at least three different categories, and selecting, for each category, the variables of the given user that are significant by considering the variables that better characterize the given body movement condition or disorder from the generated normality model;

computing, for the selected variables of the given user, a unified category score for each category; and

computing a condition or disorder score for the given user as the deviation between the computed unified category scores of the given user with the generated normality model.

8. The method of claim 7, wherein the at least three different categories comprise two additional categories, including spatial-temporal and electromyography, and wherein the monitoring sensors are selected from the group consisting of: a heart rate sensor, an electromyography sensor, an inertial sensor or goniometer, and a plantar pressure sensor.

9. The method of any one of claims 7-8, wherein the memory or database stores both the control variables and the pathology variables, and wherein the statistical-based feature selection process comprises:

comparing the control variables between two different sessions and considering the control variables that exhibited a same underlying distribution for both sessions as robust control variables; and

comparing the pathology variables between two different sessions and considering the pathology variables that exhibited a same underlying distribution for both sessions as robust pathology variables.

10. The method of claim 9, wherein the statistical-based feature selection process further comprises comparing the robust control variables with the robust pathology variables, obtaining a compared set of robust variables as a result, and selecting from the compared set, the variables showing a difference lower than a given threshold as the variables that better characterize the given body movement condition or disorder.

11. The method of any one of the previous claims 9-10, wherein the memory or database further has stored therein one or more control mobility metrics and one or more pathology mobility metrics obtained from the healthy and unhealthy users, respectively, during said different sessions, the method further comprising validating the computed condition or disorder score by comparing it with a normalized score obtained from both the one or more control mobility metrics and the one or more pathology mobility metrics.

12. The method of any one of the previous claims 7-11, wherein the specified sensor configuration comprises a time-synchronization with the other monitoring sensors.

13. The method of any one of the previous claims 7-12, wherein the cardiorespiratory function test comprises at least one of: moving the arms, getting up and sitting down from a seat, a 6-Minute Walking Test, a 10-Meter Walking Test, a Timed Up and Go Test, a Stair Climb Test.

14. A non-transitory computer-readable medium storing instructions configured to cause at least one processor of a computing system to perform the method according to claim 7.

**Patentansprüche**

1. System zur Beurteilung von Zuständen oder Störungen der Körperbewegung, umfassend:

mehrere Überwachungssensoren, von denen jeder angepasst und konfiguriert ist, um an einem unterschiedlichen Bereich des Körpers einer Person angebracht zu werden, um eine oder mehrere Variablen des Bereichs gemäß einer spezifizierten Sensorkonfiguration zu erhalten, wobei die eine oder die mehreren Variablen mindestens eine von biomechanischen oder physiologischen Variablen beinhalten;

einen Speicher oder eine Datenbank, in der mindestens eine der Folgenden gespeichert ist:

Kontrollvariablen, die während unterschiedlicher Sitzungen gleicher oder unterschiedlicher Dauer von gesunden Nutzern erhalten werden, während sie einen Funktionstest unter Verwendung der Überwachungssensoren durchführen, und

Pathologievariablen, die während unterschiedlicher Sitzungen gleicher oder unterschiedlicher Dauer von nicht gesunden Benutzern erhalten werden, während sie den Funktionstest unter Verwendung der Überwachungssensoren durchführen, wobei die nicht gesunden Benutzer an einem gegebenen Zustand oder einer gegebenen Störung der Körperbewegung leiden; und

eine Verarbeitungseinheit, die betriebsmäßig mit dem Speicher oder der Datenbank verbunden ist;
**dadurch gekennzeichnet, dass**:

die Überwachungssensoren mindestens drei Sensoren umfassen, die ausgewählt sind aus der Gruppe bestehend aus: einem Herzfrequenzsensor, einem Trägheitssensor oder einem Goniometer und einem Fußsohlendrucksensor;
wobei die Kontrollvariablen, die Pathologievariablen oder beide für jede Sitzung klassifiziert in mindestens drei unterschiedlichen Kategorien gespeichert werden, die kardiologische, kinematische und fußsohlendruckbezogene beinhalten; und
wobei die Verarbeitungseinheit zu Folgendem ausgebildet ist:

Generieren eines Normalitätsmodells für den gegebenen Zustand oder die gegebene Störung der Körperbewegung durch Implementieren eines auf Statistik basierenden Merkmalsauswahlprozesses auf den gespeicherten Kontroll- und/oder Pathologievariablen, wobei das generierte Normalitätsmodell eine einheitliche Kategoriebewertung für jede Kategorie der mindestens drei unterschiedlichen Kategorien definiert, wobei jede einheitliche Kategoriebewertung die Variablen umreißt, die den gegebenen Zustand oder die gegebene Störung der Körperbewegung besser charakterisieren;
Erlangen von Variablen, die von einem gegebenen Benutzer erhalten werden, während der gegebene Benutzer den Funktionstest während einer gegebenen Sitzung unter Verwendung der Überwachungssensoren durchführt, wobei der gegebene Benutzer unter dem gegebenen Zustand oder der gegebenen Störung der Körperbewegung leidet;
Klassifizieren der erlangten Variablen des gegebenen Benutzers in mindestens drei unterschiedliche Kategorien und Auswahl der Variablen des gegebenen Benutzers für jede Kategorie, die signifikant sind, indem die Variablen, die den gegebenen Zustand oder die gegebene Störung der Körperbewegung besser charakterisieren, aus dem generierten Normalitätsmodell berücksichtigt werden;
Berechnen einer einheitlichen Kategoriebewertung für jede Kategorie für die ausgewählten Variablen des gegebenen Benutzers; und
Berechnen einer Zustands- oder Störungsbewertung für den gegebenen Benutzer als Abweichung zwischen den berechneten einheitlichen Kategoriebewertungen des gegebenen Benutzers und dem generierten Normalitätsmodell.

2. System nach Anspruch 1, wobei die mindestens drei unterschiedlichen Kategorien zwei zusätzliche Kategorien umfassen, die räumlich-zeitliche und elektromyographiebezogene beinhalten, und wobei die Überwachungssensoren ausgewählt sind aus der Gruppe bestehend aus: einem Herzfrequenzsensor, einem Elektromyographiesensor, einem Trägheitssensor oder einem Goniometer und einem Fußsohlendrucksensor.

3. System nach einem der vorhergehenden Ansprüche, wobei der Speicher oder die Datenbank konfiguriert ist, um sowohl die Kontrollvariablen als auch die Pathologievariablen zu speichern, und wobei der auf Statistik basierende Merkmalsauswahlprozess Folgendes umfasst:

Vergleichen der Kontrollvariablen zwischen zwei unterschiedlichen Sitzungen und Berücksichtigen der Kontrollvariablen, die für beide Sitzungen dieselbe zugrunde liegende Verteilung aufweisen, als robuste Kontrollvariablen; und
Vergleichen der Pathologievariablen zwischen zwei unterschiedlichen Sitzungen und Berücksichtigen der Pathologievariablen, die für beide Sitzungen dieselbe zugrunde liegende Verteilung aufweisen, als robuste Pathologievariablen.

4. System nach Anspruch 3, wobei der auf Statistik basierende Merkmalsauswahlprozess ferner Vergleichen der robusten Kontrollvariablen mit den robusten Pathologievariablen, Erhalten eines verglichenen Satzes robuster Variablen als Ergebnis, und Auswählen aus dem verglichenen Satz der Variablen, die eine Differenz kleiner als ein gegebener Schwellenwert zeigen, als die Variablen, die den gegebenen Zustand oder die gegebene Störung der Körperbewegung besser charakterisieren, umfasst.

5. System nach einem der vorhergehenden Ansprüche, wobei die spezifizierte Sensorkonfiguration eine Zeitsynchronisation mit den anderen Überwachungssensoren umfasst.

6. System nach einem der vorhergehenden Ansprüche, wobei der Funktionstest mindestens eines von Folgendem umfasst: Bewegen der Arme, Aufstehen und Hinsetzen von einem Sitz, einen 6-Minuten-Gehtest, einen 10-Meter-Gehtest, einen Timed "Up and Go"-Test und einen Treppensteig-Test.

7. Computerimplementiertes Verfahren zur Beurteilung von Zuständen oder Störungen der Körperbewegung, wobei ein Speicher oder eine Datenbank auf ihnen gespeichert mindestens eines von Folgendem umfasst:

Kontrollvariablen, die von gesunden Nutzern während unterschiedlicher Sitzungen gleicher oder unterschiedlicher Dauer erhalten werden, während sie einen Funktionstest unter Verwendung mehrerer Überwachungssensoren durchführen, und

Pathologievariablen, die von nicht gesunden Nutzern während unterschiedlicher Sitzungen gleicher oder unterschiedlicher Dauer erhalten werden, während sie den Funktionstest unter Verwendung der mehreren Überwachungssensoren durchführen, wobei die nicht gesunden Benutzer an einem gegebenen Zustand oder einer gegebenen Störung der Körperbewegung leiden,

wobei jeder der unterschiedlichen Überwachungssensoren für jede Sitzung und für jeden gesunden und nicht gesunden Benutzer angepasst und konfiguriert ist, um an einem unterschiedlichen Bereich des Körpers angebracht zu werden, um eine oder mehrere Variablen des Bereichs gemäß einer spezifizierten Sensorkonfiguration zu erhalten, wobei die eine oder die mehreren Variablen mindestens eine von biomechanischen oder physiologischen Variablen beinhalten;

**dadurch gekennzeichnet, dass**:

die Überwachungssensoren mindestens drei Sensoren umfassen, die ausgewählt sind aus der Gruppe bestehend aus: einem Herzfrequenzsensor, einem Trägheitssensor oder einem Goniometer und einem Fußsohlendrucksensor;

wobei die Kontrollvariablen, die Pathologievariablen oder beide für jede Sitzung klassifiziert in mindestens drei unterschiedlichen Kategorien gespeichert werden, die kardiologische, kinematische und fußsohlendruckbezogene beinhalten;

und dass das Verfahren Durchführen der folgenden Schritte durch einen oder mehrere Prozessoren einer Verarbeitungseinheit umfasst:

Generieren eines Normalitätsmodells für den gegebenen Zustand oder die gegebene Störung der Körperbewegung durch Implementieren eines auf Statistik basierenden Merkmalsauswahlprozesses auf den gespeicherten Kontroll- und/oder Pathologievariablen, wobei das generierte Normalitätsmodell eine einheitliche Kategoriebewertung für jede Kategorie der mindestens drei unterschiedlichen Kategorien definiert, wobei jede einheitliche Kategoriebewertung die Variablen umreißt, die den gegebenen Zustand oder die gegebene Störung der Körperbewegung besser charakterisieren;

Erlangen, sobald sie im Speicher oder in der Datenbank gespeichert sind, von Variablen, die von einem gegebenen Benutzer erhalten werden, während der gegebene Benutzer den Funktionstest während einer gegebenen Sitzung unter Verwendung der Überwachungssensoren durchführt, wobei der gegebene Benutzer unter dem gegebenen Zustand oder der gegebenen Störung der Körperbewegung leidet;

Klassifizieren der erlangten Variablen des gegebenen Benutzers in mindestens drei unterschiedliche Kategorien und Auswahl der Variablen des gegebenen Benutzers für jede Kategorie, die signifikant sind, indem die Variablen, die den gegebenen Zustand oder die gegebene Störung der Körperbewegung besser charakterisieren, aus dem generierten Normalitätsmodell berücksichtigt werden;

Berechnen einer einheitlichen Kategoriebewertung für jede Kategorie für die ausgewählten Variablen des gegebenen Benutzers; und

Berechnen einer Zustands- oder Störungsbewertung für den gegebenen Benutzer als Abweichung zwischen den berechneten einheitlichen Kategoriebewertungen des gegebenen Benutzers und dem generierten Normalitätsmodell.

8. Verfahren nach Anspruch 7, wobei die mindestens drei unterschiedlichen Kategorien zwei zusätzliche Kategorien umfassen, die räumlich-zeitliche und elektromyographiebezogene beinhalten, und wobei die Überwachungssensoren ausgewählt sind aus der Gruppe bestehend aus: einem Herzfrequenzsensor, einem Elektromyographiesensor, einem Trägheitssensor oder Goniometer und einem Fußsohlendrucksensor.

**9.** Verfahren nach einem der Ansprüche 7-8, wobei der Speicher oder die Datenbank sowohl die Kontrollvariablen als auch die Pathologievariablen speichert, und wobei der auf Statistik basierende Merkmalsauswahlprozess Folgendes umfasst:

Vergleichen der Kontrollvariablen zwischen zwei unterschiedlichen Sitzungen und Berücksichtigen der Kontrollvariablen, die für beide Sitzungen dieselbe zugrunde liegende Verteilung aufweisen, als robuste Kontrollvariablen; und
Vergleichen der Pathologievariablen zwischen zwei unterschiedlichen Sitzungen und Berücksichtigen der Pathologievariablen, die für beide Sitzungen dieselbe zugrunde liegende Verteilung aufweisen, als robuste Pathologievariablen.

**10.** Verfahren nach Anspruch 9, wobei der auf Statistik basierende Merkmalsauswahlprozess ferner Vergleichen der robusten Kontrollvariablen mit den robusten Pathologievariablen, Erhalten eines verglichenen Satzes robuster Variablen als Ergebnis, und Auswählen aus dem verglichenen Satz der Variablen, die eine Differenz kleiner als ein gegebener Schwellenwert zeigen, als die Variablen, die den gegebenen Zustand oder die gegebene Störung der Körperbewegung besser charakterisieren, umfasst.

**11.** Verfahren nach einem der vorhergehenden Ansprüche 9-10, wobei der Speicher oder die Datenbank ferner eine oder mehrere Kontrollmobilitätsmetriken und eine oder mehrere Pathologiemobilitätsmetriken gespeichert hat, die während der unterschiedlichen Sitzungen jeweils von den gesunden und nicht gesunden Benutzern erhalten werden, wobei das Verfahren ferner Validieren der berechneten Zustands- oder Störungsbewertung durch Vergleichen mit einer normalisierten Bewertung umfasst, die sowohl aus der einen oder den mehreren Kontrollmobilitätsmetriken als auch aus der einen oder den mehreren Pathologiemobilitätsmetriken erhalten wird.

**12.** Verfahren nach einem der vorhergehenden Ansprüche 7-11, wobei die spezifizierte Sensorkonfiguration eine Zeitsynchronisation mit den anderen Überwachungssensoren umfasst.

**13.** Verfahren nach einem der vorhergehenden Ansprüche 7-12, wobei der kardiorespiratorische Funktionstest mindestens eines von Folgendem umfasst: Bewegen der Arme, Aufstehen und Hinsetzen von einem Sitz, einen 6-Minuten-Gehtest, einen 10-Meter-Gehtest, einen Timed "Up and Go"-Test, einen Treppensteig-Test.

**14.** Nicht-transitorisches computerlesbares Medium, das Anweisungen speichert, die konfiguriert sind, um mindestens einen Prozessor eines Computersystems zu veranlassen, das Verfahren nach Anspruch 7 durchzuführen.

## Revendications

**1.** Système pour l'évaluation des affections ou des troubles du mouvement corporel, comprenant :

plusieurs capteurs de surveillance, chacun étant adapté et configuré pour être attaché à une zone différente du corps d'une personne pour obtenir une ou plusieurs variables de ladite zone selon une configuration de capteur spécifiée, l'une ou plusieurs variables comportant au moins une parmi des variables biomécaniques et physiologiques ;
une mémoire ou base de données, ayant stockées dans celle-ci au moins une parmi :

des variables de contrôle, obtenues pendant différentes sessions de durée identique ou différente à partir d'utilisateurs sains pendant qu'ils effectuent un test fonctionnel en utilisant les capteurs de surveillance, et
des variables de pathologie, obtenues pendant différentes sessions de durée identique ou différente à partir d'utilisateurs malades pendant qu'ils effectuent le test fonctionnel en utilisant les capteurs de surveillance, les utilisateurs malades souffrant d'une affection ou trouble du mouvement corporel donnée ; et

une unité de traitement reliée de manière opérationnelle à ladite mémoire ou base de données ;
**caractérisé en ce que** :

les capteurs de surveillance comprennent au moins trois capteurs qui sont choisis dans qui sont choisis dans le groupe constitué par : un capteur de fréquence cardiaque, un capteur inertiel ou un goniomètre, et un capteur de pression plantaire ;
les variables de contrôle, les variables de pathologie, ou les deux, pour chaque session, sont stockées classées dans au moins trois catégories différentes, y compris cardiaque, cinématique et de pression plantaire ; et

l'unité de traitement est configurée pour :

générer un modèle de normalité pour ladite affection ou trouble du mouvement corporel donnée en mettant en œuvre un procédé de sélection de caractéristiques en fonction de statistiques sur les variables de contrôle et/ou de pathologie stockées, le modèle de normalité généré définissant un score de catégorie unifié pour chaque catégorie desdites au moins trois catégories différentes, chaque score de catégorie unifié décrivant les variables qui caractérisent le mieux l'affection ou trouble du mouvement corporel donnée ;

obtenir des variables obtenus à partir d'un utilisateur donné pendant que l'utilisateur donné effectue le test fonctionnel pendant une session donnée en utilisant les capteurs de surveillance, l'utilisateur donné souffrant de l'affection ou trouble du mouvement corporel donnée ;

classer les variables obtenues de l'utilisateur donné dans les au moins trois catégories différentes, et sélectionner, pour chaque catégorie, les variables de l'utilisateur donné qui sont significatives en prenant compte des variables qui caractérisent le mieux l'affection ou trouble du mouvement corporel donnée à partir du modèle de normalité généré ;

calculer, pour les variables sélectionnées de l'utilisateur donné, un score de catégorie unifié pour chaque catégorie ; et

calculer un score d'affection ou trouble pour l'utilisateur donné comme l'écart entre les scores de catégorie unifiés calculés de l'utilisateur donné et le modèle de normalité généré.

2. Système selon la revendication 1, dans lequel les au moins trois catégories différentes comprennent deux catégories additionnelles, y compris spatio-temporelle et électromyographique, et dans lequel les capteurs de surveillance sont choisis dans le groupe constitué par : un capteur de fréquence cardiaque, un capteur d'électromyographie, un capteur inertiel ou un goniomètre, et un capteur de pression plantaire.

3. Système selon l'une quelconque des revendications précédentes, dans lequel la mémoire ou base de données est configurée pour stocker aussi bien les variables de contrôle que les variables de pathologie, et dans lequel le procédé de sélection de caractéristiques en fonction de statistiques comprend :

comparer les variables de contrôle entre deux sessions différentes et prendre compte des variables de contrôle qui montrent une même distribution sous-jacente pour les deux sessions comme des variables de contrôle robustes ; et

comparer les variables de pathologie entre deux sessions différentes et prendre compte des variables de pathologie qui montrent une même distribution sous-jacente pour les deux sessions comme des variables de pathologie robustes.

4. Système selon la revendication 3, dans lequel le procédé de sélection de caractéristiques en fonction de statistiques comprend en outre comparer les variables de contrôle robustes avec les variables de pathologie robustes, obtenir comme résultat un ensemble comparé de variables robustes, et sélectionner, à partir de l'ensemble comparé, les variables qui montrent une différence inférieure à un seuil donné comme les variables qui caractérisent le mieux l'affection ou trouble du mouvement corporel donnée.

5. Système selon l'une quelconque des revendications précédentes, dans lequel la configuration de capteur spécifiée comprend une synchronisation dans le temps avec les autres capteurs de surveillance.

6. Système selon l'une quelconque des revendications précédentes, dans lequel le test fonctionnel comprend au moins un parmi : bouger les bras, se lever et s'asseoir dans un siège, un test de marche de 6 minutes, un test de marche de 10 mètre, un test chronométré du lever de chaise, et un test de montée d'escalier.

7. Procédé mis en œuvre par ordinateur pour l'évaluation des affections ou des troubles du mouvement corporel, dans lequel une mémoire ou base de données comprend stockées dans celle-ci au moins une parmi :

des variables de contrôle, obtenues à partir d'utilisateurs sains pendant qu'ils effectuent un test fonctionnel en utilisant plusieurs capteurs de surveillance pendant différentes sessions de durée identique ou différente, et des variables de pathologie, obtenues à partir d'utilisateurs malades pendant qu'ils effectuent le test fonctionnel en utilisant les plusieurs capteurs de surveillance pendant différentes sessions de durée identique ou différente, les utilisateurs malades souffrant d'une affection ou trouble du mouvement corporel donnée,

chacun des différents capteurs de surveillance, pour chaque session et pour chaque utilisateur sain et malade, étant adapté et configuré pour être attaché à une zone différente du corps pour obtenir une ou plusieurs variables de ladite zone selon une configuration de capteur spécifiée, l'une ou plusieurs variables comportant au moins une parmi des variables biomécaniques et physiologiques ;

**caractérisé en ce que** :

les capteurs de surveillance comprennent au moins trois capteurs qui sont choisis dans le groupe constitué par : un capteur de fréquence cardiaque, un capteur inertiel ou un goniomètre, et un capteur de pression plantaire ;

les variables de contrôle, les variables de pathologie, ou les deux, pour chaque session, sont stockées classées dans au moins trois catégories différentes, y compris cardiaque, cinématique et de pression plantaire ;

et **en ce que** le procédé comprend la réalisation, par un ou plusieurs processeurs d'une unité de traitement, des étapes suivantes :

générer un modèle de normalité pour ladite affection ou trouble du mouvement corporel donnée en mettant en œuvre un procédé de sélection de caractéristiques en fonction de statistiques sur les variables de contrôle et/ou de pathologie stockées, le modèle de normalité généré définissant un score de catégorie unifié pour chaque catégorie desdites au moins trois catégories différentes, chaque score de catégorie unifié décrivant les variables qui caractérisent le mieux l'affection ou trouble du mouvement corporel donnée ;

obtenir, une fois stockées dans la mémoire ou base de données, des variables obtenues à partir d'un utilisateur donné pendant que l'utilisateur donné effectue le test fonctionnel pendant une session donnée en utilisant les capteurs de surveillance, l'utilisateur donné souffrant de l'affection ou trouble du mouvement corporel donnée ;

classer les variables obtenues de l'utilisateur donné dans les au moins trois catégories différentes, et sélectionner, pour chaque catégorie, les variables de l'utilisateur donné qui sont significatives en prenant compte des variables qui caractérisent le mieux l'affection ou trouble du mouvement corporel donnée à partir du modèle de normalité généré ;

calculer, pour les variables sélectionnées de l'utilisateur donné, un score de catégorie unifié pour chaque catégorie ; et

calculer un score d'affection ou trouble pour l'utilisateur donné comme l'écart entre les scores de catégorie unifiés calculés de l'utilisateur donné et le modèle de normalité généré.

8. Procédé selon la revendication 7, dans lequel les au moins trois catégories différentes comprennent deux catégories additionnelles, y compris spatio-temporelle et électromyographique, et dans lequel les capteurs de surveillance sont choisis dans le groupe constitué par : un capteur de fréquence cardiaque, un capteur d'électromyographie, un capteur inertiel ou goniomètre, et un capteur de pression plantaire.

9. Procédé selon l'une quelconque des revendications 7-8, dans lequel la mémoire ou base de données stocke aussi bien les variables de contrôle que les variables de pathologie, et dans lequel le procédé de sélection de caractéristiques en fonction de statistiques comprend :

comparer les variables de contrôle entre deux sessions différentes et prendre compte des variables de contrôle qui montrent une même distribution sous-jacente pour les deux sessions comme des variables de contrôle robustes ; et

comparer les variables de pathologie entre deux sessions différentes et prendre compte des variables de pathologie qui montrent une même distribution sous-jacente pour les deux sessions comme des variables de pathologie robustes.

10. Procédé selon la revendication 9, dans lequel le procédé de sélection de caractéristiques en fonction de statistiques comprend en outre comparer les variables de contrôle robustes avec les variables de pathologie robustes, obtenir comme résultat un ensemble comparé de variables robustes, et sélectionner, à partir de l'ensemble comparé, les variables qui montrent une différence inférieure à un seuil donné comme les variables qui caractérisent le mieux l'affection ou trouble du mouvement corporel donnée.

11. Procédé selon l'une quelconque des revendications précédentes 9-10, dans lequel la mémoire ou base de données

EP 4 511 847 B1

présente en outre, stockées dans celle-ci, une ou plusieurs mesures de mobilité de contrôle et une ou plusieurs mesures de mobilité de pathologie obtenues à partir des utilisateurs sains et malades, respectivement, pendant lesdites différentes sessions, le procédé comprenant en outre la validation du score d'affection ou trouble calculé par comparaison de celui-ci avec un score normalisé obtenu à partir aussi bien de l'une ou plusieurs mesures de mobilité de contrôle que de l'une ou plusieurs meures de mobilité de pathologie.

12. Procédé selon l'une quelconque des revendications précédentes 7-11, dans lequel la configuration de capteur spécifiée comprend une synchronisation dans le temps avec les autres capteurs de surveillance.

13. Procédé selon l'une quelconque des revendications précédentes 7-12, dans lequel le test fonctionnel cardiorespiratoire comprend au moins un parmi : bouger les bras, se lever et s'asseoir dans un siège, un test de marche de 6 minutes, un test de marche de 10 mètres, un test chronométré du lever de chaise, un test de montée d'escalier.

14. Support non transitoire lisible par ordinateur stockant des instructions configurées pour amener au moins un processeur d'un système informatique à réaliser le procédé selon la revendication 7.

18

Fig. 1

2001C — Session data Control group

Session data Pathology group — 2001P

2002C — Control biomechanical and/or physiological variables

Pathology biomechanical and/or physiological variables — 2002P

2003C — Data cleansing

Data cleansing — 2003P

2004C — Feature selection I

Feature selection I — 2004P

Feature selection II — 2005

Generation of unified score — 2006

Normality Model for condition/disorder X — 2007

# Fig. 2

Spatio-temporal    Cardiac    Kinematics    EMG    Plantar pressure

DMD    CONTROL    DMD    CONTROL    DMD    CONTROL    DMD    CONTROL    DMD    CONTROL

# Fig. 3

# Fig. 4

# Fig. 5

Fig. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021064195 A1

**Non-patent literature cited in the description**

- **SCHOLLHORN W I ED-GEFEN AMIT**. *Applications of artificial neural nets in clinical biomechanics*